(19) Europäisches Patentamt
European Patent Office
Office européen des brevets

(11) Veröffentlichungsnummer : **0 300 968 B1**

# (12) EUROPÄISCHE PATENTSCHRIFT

(45) Veröffentlichungstag der Patentschrift : **11.12.91 Patentblatt 91/50**

(51) Int. Cl.[5] : **C07C 275/54**, C07C 205/37, C07C 217/84, A01N 47/34

(21) Anmeldenummer : **88810483.3**

(22) Anmeldetag : **15.07.88**

(54) **N-Benzoyl-N'-2,5-dihalogen-4-perfluoro-alkoxyphenylharnstoffe, ihre Herstellung und Verwendung bei der Kontrolle von Schädlingen.**

(30) Priorität : **21.07.87 CH 2751/87**
**20.05.88 CH 1920/88**

(43) Veröffentlichungstag der Anmeldung : **25.01.89 Patentblatt 89/04**

(45) Bekanntmachung des Hinweises auf die Patenterteilung : **11.12.91 Patentblatt 91/50**

(84) Benannte Vertragsstaaten : **BE CH DE FR GB IT LI NL**

(56) Entgegenhaltungen :
**EP-A- 0 071 279**
**EP-A- 0 179 021**

(56) Entgegenhaltungen :
**EP-A- 0 194 688**
**EP-A- 0 226 642**
**EP-A- 0 231 152**
**EP-A- 0 235 089**
**DD-A- 253 614**
**US-A- 4 518 804**
**US-A- 4 536 341**

(73) Patentinhaber : **CIBA-GEIGY AG**
**Klybeckstrasse 141**
**CH-4002 Basel (CH)**

(72) Erfinder : **Drabek, Jozef, Dr.**
**Benkenstrasse 12**
**CH-4104 Oberwil (CH)**
Erfinder : **Siegrist, Urs**
**Rebenweg 32**
**CH-5268 Eiken (CH)**

**EP 0 300 968 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue substituierte N-Benzoyl-N'-2,5-dihalogen-4-perfluoralkoxyphenylharnstoffe, Verfahren und Zwischenprodukte zu ihrer Herstellung und ihre Verwendung in der Schädlingsbekämpfung.

Die erfindungsgemässen Verbindungen entsprechen der Formel I

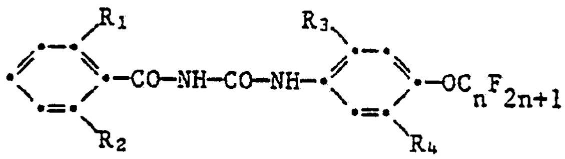

(I),

worin $R_1$ für Wasserstoff, Fluor, Chlor oder Brom ; $R_2$ für Fluor oder Chlor ; $R_3$ und $R_4$ für Fluor, Chlor oder Brom; und n für 2, 3, 4, 5, 6, 7 oder 8 stehen, mit der Massgabe, dass $R_4$ Fluor oder Chlor bedeutet, wenn gleichzeitig $R_3$ für Fluor und n für die Zahl 2 stehen.

Die als Substituenten in Betracht kommenden perfluorierten $C_2$-$C_8$-Alkyle können geradkettig oder verzweigt sein, sind aber vorzugsweise unverzweigt. Geeignete Beispiele solcher Substituenten sind u.a. $CF_2CF_3$, $(CF_2)_2CF_3$, $(CF_2)_3CF_3$, $CF_2CF(CF_3)_2$ oder die Perfluorpentyle, -hexyle, -heptyle oder -octyle.

Unter den Verbindungen der Formel I stehen diejenigen im Vordergrund, in denen $R_1$ für Wasserstoff, Fluor oder Chlor ; $R_2$ für Fluor oder Chlor ; $R_3$ für Fluor oder Chlor ; $R_4$ für Fluor, Chlor oder Brom ; und n für 2, 3, 4, 5, 6 oder 7 stehen.

Dabei sind diejenigen Verbindungen der Formel I bevorzugt, in denen $R_1$ für Wasserstoff oder Fluor ; $R_2$ für Fluor oder Chlor ; $R_3$ und $R_4$ je für Chlor ; und n für 2, 3 oder 4 stehen.

Die erfindungsgemässen Verbindungen können nach im Prinzip bekannten Verfahren hergestellt werden. Solche Verfahren sind u.a. in den DE-OS 2123236, 2601780 und 3240975 beschrieben. So können die Verbindungen der Formel I z.B. erhalten werden, indem man

a) ein Anilin der Formel II

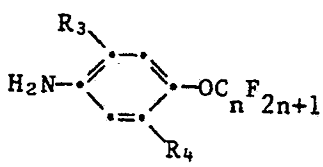

(II)

mit einem Benzoylisocyanat der Formel III

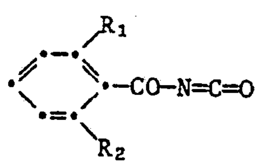

(III),

oder

b) ein Isocyanat der Formel IV

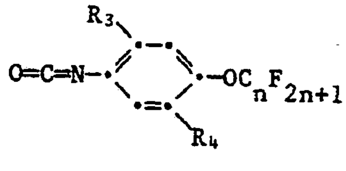

(IV)

mit einem Benzamid der Formel V

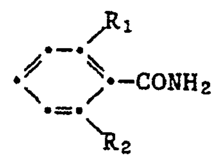 (V),

oder

c) ein Anilin der Formel II mit einem Urethan der Formel VI

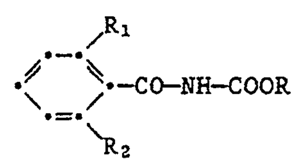 (VI)

umsetzt. In den obigen Formeln II bis VI haben die Symbole $R_1$, $R_2$, $R_3$, $R_4$ und n die für Formel I angegebene Bedeutung und R steht für einen $C_1$-$C_8$-Alkylrest, der gegebenenfalls mit Halogen, vorzugsweise Chlor, substituiert sein kann.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.b. Aether und ätherartige Verbindungen, wie Diäthyläther, Dipropyläther, Dibutyläther, Dioxan, Dimethoxyäthan und Tetrahydrofuran ; N,N-dialkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid sowie Ketone, z.B. aceton, Methyläthylketon, Methylisopropylketon und Methylisobutylketon. Verfahren a) wird im allgemeinen bei einer Temperatur von −10 bis +200°C, vorzugsweise zwischen 0 und +100°C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis +150°C, vorzugsweise beim Siedepunkt des verwendeten Lösungsmittels, und gegebenenfalls in Gegenwart einer organischen Base, wie Pyridin, und/oder unter Zusatz eines Alkali-oder Erdalkalimetalls, vorzugsweise Natrium. Für das Verfahren c), d.h. für die Umsetzung der Urethane der Formel VI mit einem Anilin der Formel II, werden Temperaturen zwischen etwa+60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III, V und VI sind teilweise bekannt. Sowohl die bekannten als auch die gegebenenfalls neuen Ausgangsstoffe können analog bekannten Verfahren hergestellt werden.

Bei den Ausgangsstoffen der Formel II handelt es sich um neuartige Verbindungen, die ebenfalls einen Gegenstand der vorliegenden Erfindung bilden. Die Verbindungen der Formel II können in an sich bekannter Weise z.B. dadurch hergestellt werden, dass man entsprechend substituierte Nitrobenzole der Formel VII

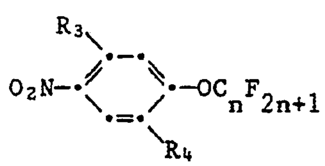 (VII)

in Analogie zu dem in J. Org. Chem. 29 (1964), 1, aufgezeigten Verfahren hydriert (vgl. auch die dort zitierte Literatur). Aber auch durch chemische Reduktion (z.B. mittels Sn-(II)-Chlorid/HCl) der entsprechenden Nitroverbindungen der Formel VII sind Aniline der Formel II zugänglich (Vgl. Houben Weyl, "Methoden d. org. Chemie" 11/1, 422).

Die Nitroverbindungen der Formel VII sind ebenfalls neu und bilden einen Gegenstand der vorliegenden Erfindung. Sie sind nach im Prinzip bekannten Methoden herstellbar, z.B. durch Fluorierung von Verbindungen der Formel VIII

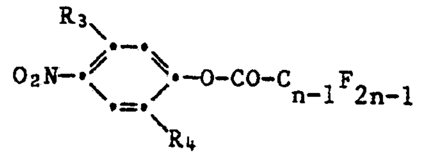

(VIII)

mittels $SF_4$ und HF (vgl. z.B. Organic Reactions Vol. 21, p. 37-42, 1974 ; Vol. 34, p. 339, 1985). Die Verbindungen der Formel VIII sind bekannt oder können nach im Prinzip bekannten Methoden hergestellt werden.

Zu Benzoylisocyanaten gemäss Formel III kann man unter anderem wie folgt gelangen (vgl. J. Agr. Food Chem. 21, 348 und 993 ; 1973) :

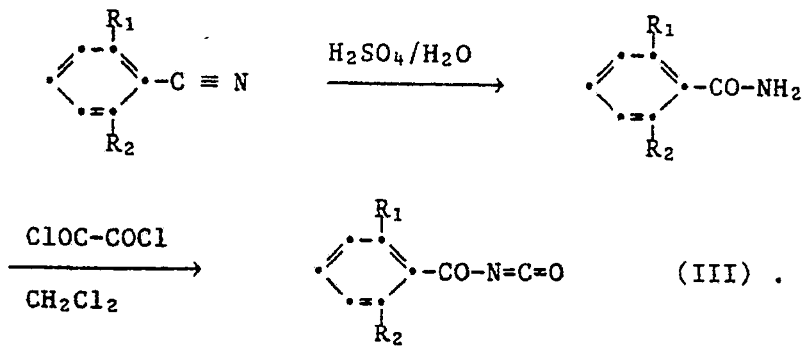


(III) .

Die 4-Perfluoralkoxy-phenylisocyanate der Formel IV lassen sich z.B. durch Phosgenierung der Aniline der Formel II nach allgemein üblichen Verfahren herstellen. Die weiterhin als Ausgangsstoffe zu verwendenden Benzamide der Formel V sind bekannt (vgl. z.B. Beilstein "Handbuch der organischen Chemie" Bd. 9, S. 336).

Urethane der Formel VI können nach im Prinzip bekannten Methoden erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure Cl-COOR.

Aus der US Patentschrift 4,518,804 sind ganz allgemein N-(2,6-Dihalogenbenzoyl)-N'-(2,5-dichlor-4-halogenalkoxyphenyl)-harnstoffe mit insektizider Wirkung bekannt. Perfluorierte Alkoxyreste werden jedoch nicht offenbart.

Ueberraschenderweise wurde gefunden, dass die erfindungsgemässen Verbindungen bei günstiger Warmblüter- und Pflanzenverträglichkeit wertvolle Wirkstoffe in der Schädlingsbekämpfung sind. So eignen sich die Verbindungen der Formel I z.b. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thyusanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht. Ferner eignen sich die Verbindungen der Formel I zur Bekämpfung von Schnecken in Zier- und Nutzpflanzungen.

Die gute pestizide Wirkung der erfindungsgemässen Verbindungen der Formel I entspricht einer Abtötungsrate (Mortalität) von mindestens 50-60% der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht : Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensor-

bitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99%, insbesondere 0,1 bis 95%, Wirkstoff der Formel I oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1 : Herstellung

1.1. Zwischenprodukte

1.1.1. Nitrobenzole

1.1.1.1. 2,5-Dichlor-4-pentafluoräthoxy-nitrobenzol

Ein Autoklav wird mit 20 g 2,5-Dichlor-4-nitrophenyl-trifluoracetat beschickt und evakuiert. Bei −10 bis 0°C werden zuerst 60 g Fluorwasserstoff zugegeben und dann 14,3 g Schwefeltetrafluorid mit Inertgas portionenweise eingepresst. Anschliessend wird das Reaktionsgemisch 15 Stunden lang bei+50°C gerührt. Danach wird der leichtflüchtige Anteil bei Raumtemperatur abdestilliert, der Rückstand in Methylenchlorid aufgenommen und in Eiswasser gegossen. Die organische Phase wird abgetrennt, die wässrige Phase zweimal mit Methylenchlorid extrahiert, und schliesslich werden die vereinigten organischen Extrakte je zweimal mit Wasser und Natriumcarbonat gewaschen und über Natriumsulfat getrocknet. Das Lösungsmittel wird abgedampft, der Rückstand in einem Gemisch Hexan/Aether (30 : 1) aufgenommen und über Kieselgel filtriert. Nach abdestillieren des Lösungsmittels liegt die Titelverbindung der Formel

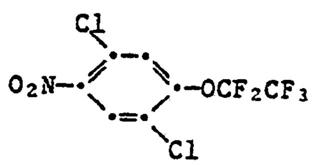

(Verb. 1.1.1.1.)

als hellgelbes Oel vor ; $n_D^{22}$ : 1,4678 ; Sdp. 65-68°C/0,13 mbar (0,1 Torr).

Auf analoge Weise werden die folgenden Verbindungen hergestellt :

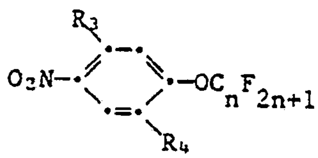

| Verb. Nr. | $R_3$ | $R_4$ | $C_nF_{2n+1}$ | phys. Daten |
|---|---|---|---|---|
| 1.1.1.2. | Cl | Cl | $(CF_2)_2CF_3$ | Sdp. 74-77°C/0,2 mbar (0,15 Torr) |
| 1.1.1.3. | Cl | Cl | $(CF_2)_3CF_3$ | Sdp. 76-82°C/0,07 mbar (0,05 Torr) |
| 1.1.1.4. | F | F | $(CF_2)_2CF_3$ | Sdp. 55-58°C/0,07 mbar (0,05 Torr) |

## 1.1.2 Aniline

### 1.1.2.1 2,5-Dichlor-4-pentafluoräthoxy-anilin

41 g 2,5-Dichlor-4-pentafluoräthoxy-nitrobenzol werden in 410 ml Tetrahydrofuran gelöst und bei Raumtemperatur während 32 Stunden in Gegenwart von Raney-Nickel hydriert (H2-Aufnahme: 7,62 1). Das Reaktionsgemisch wird filtriert und eingeengt und der Rückstand destilliert. Die Titelverbindung der Formel

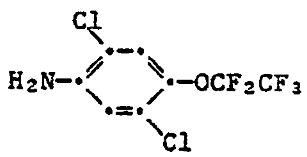

(Verb. 1.1.2.1.)

liegt als farblose Flüssigkeit vor; Sdp. 56-61°C/0,05 mbar (0,04 Torr).

Auf analoge Weise werden die folgenden Verbindungen hergestellt

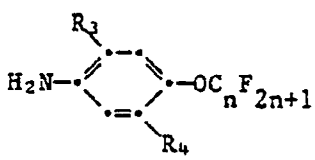

| Verb. Nr. | $R_3$ | $R_4$ | $C_nF_{2n+1}$ | phys. Daten |
|---|---|---|---|---|
| 1.1.2.2. | Cl | Cl | $(CF_2)_2CF_3$ | Sdp. 110°C/0,05 mbar (0,04 Torr) |
| 1.1.2.3. | Cl | Cl | $(CF_2)_3CF_3$ | Sdp. 140°C/0,11 mbar (0,08 Torr) |
| 1.1.2.4. | F | F | $(CF_2)_2CF_3$ | Sdp. 95-100°C/0,05 mbar (0,04 Torr) |

## 1.2 Endprodukte

### 1.2.1 N-(2,6-Difluorbenzoyl)-N'-(2,5-dichlor-4-pentafluoräthoxyphenyl)-harnstoff

5,6 g 2,5-Dichlor-4-pentafluoräthoxy-anilin, gelöst in 40 ml trockenem Toluol, werden bei Raumtemperatur unter Feuchtigkeitsausschluss mit 3,4 g 2,6-Difluorbenzoylisocyanat, gelöst in 10 ml trockenem Toluol, versetzt und 10 Stunden lang gerührt. Anschliessend werden etwa 60% des Lösungsmittels entfernt. Der entstandene Niederschlag wird abgenutscht, mit Hexan gewaschen und im Vacuum getrocknet. Die Titelverbindung der Formel

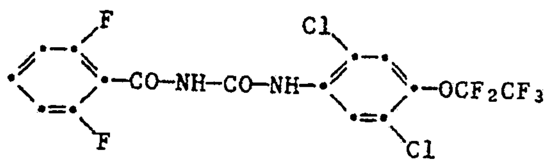 (Verb. Nr. 1.2.1.)

liegt in Form farbloser Kirstalle vor ; Smp. 178-179°C.

Auf analoge Weise werden die folgenden Verbindungen hergestellt :

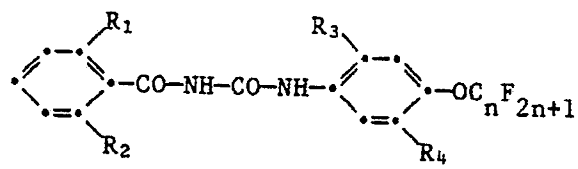

| Verb. Nr. | $R_1$ | $R_2$ | $R_3$ | $R_4$ | $C_nF_{2n+1}$ | phys. Daten |
|---|---|---|---|---|---|---|
| 1.2.2. | F | F | Cl | Cl | $(CF_2)_2CF_3$ | Smp. 176-178°C |
| 1.2.3. | F | F | Cl | Cl | $(CF_2)_3CF_3$ | Smp. 171-173°C |
| 1.2.4. | H | F | Cl | Cl | $CF_2CF_3$ | Smp. 164-165°C |
| 1.2.5. | H | F | Cl | Cl | $(CF_2)_3CF_3$ | Smp. 162-164°C |
| 1.2.6. | H | Cl | Cl | Cl | $CF_2CF_3$ | Smp. 177-179°C |
| 1.2.7. | H | Cl | Cl | Cl | $(CF_2)_2CF_3$ | Smp. 178-179°C |
| 1.2.8. | H | Cl | Cl | Cl | $(CF_2)_3CF_3$ | Smp. 166-168°C |
| 1.2.9. | F | F | F | F | $(CF_2)_2CF_3$ | Smp. 191-193°C |
| 1.2.10. | H | Cl | F | F | $(CF_2)_2CF_3$ | Smp. 168-169°C |
| 1.2.11. | H | F | F | F | $(CF_2)_2CF_3$ | Smp. 163-164°C |
| 1.2.12. | Cl | Cl | F | F | $(CF_2)_2CF_3$ | Smp. 212-213°C |
| 1.2.13 | Cl | F | F | F | $(CF_2)_2CF_3$ | Smp. 218-222°C |
| | F | F | Cl | Cl | $(CF_2)_4CF_3$ | |
| | F | F | Cl | Cl | $(CF_2)_6CF_3$ | |
| | F | F | Cl | Cl | $(CF_2)_7CF_3$ | |
| | F | F | F | Br | $(CF_2)_2CF_3$ | |
| | F | F | F | Br | $(CF_2)_6CF_3$ | |
| | F | F | F | F | $(CF_2)_6CF_3$ | |
| | H | Cl | Cl | Cl | $(CF_2)_4CF_3$ | |
| | H | Cl | Cl | Cl | $(CF_2)_6CF_3$ | |
| | H | Cl | Cl | Cl | $(CF_2)_7CF_3$ | |

Beispiel 2 : Formulierungen von Wirkstoffen der Formel I gemäss Herstellungsbeispiel 1.2.

(% = Gewichtsprozent)

2.1. Emulsions-Konzentrate

| | |
|---|---|
| Wirkstoff gemäss dem Herstellungsbeispiel 1.2. | 10 % |
| Ricinusöl-polyäthylenglykoläther (36 Mol AeO) | 25 % |
| Butanol | 15 % |
| Essigester | 50 % |

Aus solchen Konzentraten können durch Verdünnen mit Wasser Emulsionen jeder gewünschten Konzentration hergestellt werden.

| 2.2. Lösungen | a) | b) |
|---|---|---|
| Wirkstoff gemäss dem Herstellungsbeispiel 1.2. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3. Granulate | a) | b) |
|---|---|---|
| Wirkstoff gemäss dem Herstellungsbeispiel 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Extruder Granulat

| | |
|---|---|
| Wirkstoff gemäss dem Herstellungsbeispiel 1.2 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. dieses Gemisch wird extrudiert und anschliessend im Luftstrom getrocknet.

2.5. Umhüllungs-Granulat

| | |
|---|---|
| Wirkstoff gemäss dem Herstellungsbeispiel 1.2. | 3% |
| Polyäthylenglykol (MG 200) | 3% |
| Kaolin | 94% |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6. Stäubemittel | a) | b) | c) | d) |
|---|---|---|---|---|
| Wirkstoff gemäss dem Herstellungsbeispiel 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7. Spritzpulver | a) | b) | c) |
|---|---|---|---|
| Wirkstoff gemäss dem Herstellungsbeispiel 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8. Suspensions-Konzentrat

| | |
|---|---|
| Wirkstoff gemäss dem Herstellungsbeispiel 1.2. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3 : Biologische Prüfungen

3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50% Luftfeuchtigkeit belassen. Nach 24 Stunden wird die insektizide Wirkung durch Ermittlung der Abtötungsrate festgestellt.

Verbindungen gemäss Beispiel 1.2. zeigen gute Wirkung im obigen Test.

3.2. Wirkung gegen Lucilia sericata

Zu 9 ml eines Zuchtmediums wird bei 50°C 1 ml einer 0,5% Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss Beispiel 1.2. zeigen in diesem Test gute Wirkung gegen Lucilia sericata.

3.3. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1.2. zeigen gute Wirkung in diesem Test.

3.4. Insektizide Frassgift-Wirkung

Baumwollpflanzen (ca. 20 cm hoch) werden mit einer wässrigen Wirkstoffemulsion (erhalten aus einem 10%igen emulgierbaren Konzentrat) besprüht, wobei die Wirkstoffemulsion 100 ppm der zu prüfenden Verbindung enthält.

Nach dem Antrocknen des Belages werden die Baumwollpflanzen je mit Spodoptera littoralis- und Heliothis virescens-Larven im dritten larvalen Stadium besetzt. Der Versuch wird bei 24°C und 60% relativer Luftfeuchtigkeit durchgeführt. In Abständen von jeweils 24 Stunden werden Mortalität sowie Entwicklungs- und Häutungsstörungen der angesetzten Larven bestimmt.

Verbindungen gemäss Beispiel 1.2. zeigen gute Wirkung in diesem Test.

3.5. Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit :

a) 50 Larven von Spodoptera littoralis oder Heliothis virescens des ersten larvalen Stadiums ;
b) 20 Larven von Spodoptera littoralis oder Heliothis virescens des dritten larvalen Stadiums ;
c) zwei Eispiegeln von Spodoptera littoralis oder Heliothis virescens (dazu werden je 2 Blätter einer Pflanze in einem beidseitig mit Gaze verschlossenen Plexiglaszylinder eingeschlossen) ; zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien :

a) Anzahl der noch lebenden Larven,
b) larvale Entwicklungs- und Häutungshemmung,
c) Frassschaden (Schabfrass und Lochfrass),
d) Schlupfrate (Anzahl der aus den Eiern geschlüpften Larven).

Verbindungen gemäss Beispiel 1.2. zeigen in einer Konzentration von 400 ppm gute Gesamt-Wirksamkeit in obigem Test.

3.6. Ovizide Wirkung auf Spodoptera littoralis

Auf Filterpapier abgelegte Eier von Spodoptera littoralis werden aus dem Papier ausgeschnitten und in eine 0,05 gew.%ige Lösung des Wirkstoffes in einem Aceton/Wasser-Gemisch (1 : 1) getaucht. Die so behandelten Eiablagen werden dann aus diesem Gemisch herausgenommen und bei 28°C und 60% relativer Luftfeuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen gemäss Beispiel 1.2. zeigen gute Wirkung im obigen Test.

3.7. Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Beispiel 1.2. zeigen gute Wirkung in obigem Test.

3.8. Reproduktions-Beeinflussung von Anthonomus grandis

Adulte Anthonomus grandis, die nach dem Schlupf nicht älter als 24 Stunden sind, werden in Gruppen von jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird

die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen gemäss Beispiel 1.2. zeigen eine gute reproduktionsreduzierende Wirkung im obigen Test.

3.9. Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60% relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss Beispiel 1.2. zeigen gute Wirkung in diesem Test.

3.10. Wirkung gegen Epilachna varivestis

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60% relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss Beispiel 1.2. zeigen gute Wirkung im obigen Test.

3.11. Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60% relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Beispiel 1.2. zeigen in obigem Test eine 80-100%ige ovizide Wirkung (Mortalität) gegen Heliothis virescens und Spodoptera littoralis.

3.12. Frassgift-Wirkung auf Heliothis virescens-Larven ($L_1$)

Eingetopfte Sojapflanzen im 4-5-Blattstadium werden mit einer wässrigen Wirkstoffemulsion (erhalten aus einem 10%igen emulgierbaren Konzentrat) besprüht, die den Wirkstoff in einer Konzentraton von 0,8 ppm enthält.

Nach dem Antrocknen des Sprühbelages wird jede Sojapflanze mit 20 Heliothis virescens-Larven im ersten larvalen Stadium besiedelt. Der Versuch wird bei 26°C und ca. 55% relativer Luftfeuchtigkeit durchgeführt. Die Bonitur erfolgt 6 Tage nach der Besiedlung, wobei die Mortalität der Larven in Prozenten bestimmt wird.

| Verbindungen | | Mortalität in Prozenten bei 0,8 ppm |
|---|---|---|
| 1. | Verb. Nr. 1.2.1. | 70 |
| 2. | Verb. Nr. 1.2.2. | 85 |
| 3. | Verb. Nr. 1.2.3. | 100 |
| 4. | Verb. Beispiel 1B US 4,518,804* | 25 |

*

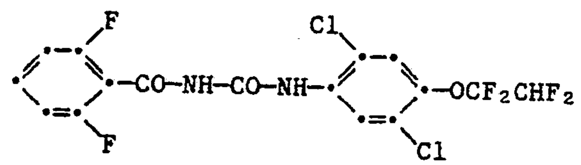

**Patentansprüche**

1. Verbindungen der Formel I

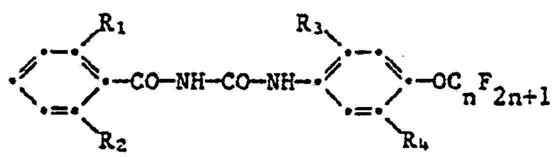

(I),

worin $R_1$ für Wasserstoff, Fluor, Chlor oder Brom ; $R_2$ für Fluor oder Chlor ; $R_3$ und $R_4$ je für Fluor, Chlor oder Brom ; und n für 2, 3, 4, 5, 6, 7 oder 8 stehen, mit der Massgabe, dass $R_4$ Fluor oder Chlor bedeutet, wenn gleichzeitig $R_3$ für Fluor und n für die Zahl 2 stehen.

2. Verbindungen der Formel I gemäss Anspruch 1, worin $R_1$ für Wasserstoff ; Fluor oder Chlor ; $R_2$ für Fluor oder Chlor ; $R_3$ für Fluor oder Chlor ; $R_4$ für Fluor, Chlor oder Brom ; und n für 2, 3, 4, 5, 6 oder 7 stehen.

3. Verbindungen der Formel I gemäss Anspruch 2, worin $R_1$ für Wasserstoff oder Fluor ; $R_2$ für Fluor oder Chlor ; $R_3$ und $R_4$ je für Chlor ; und n für 2, 3 oder 4 stehen.

4. Die Verbindungen gemäss Anspruch 3 der Formeln

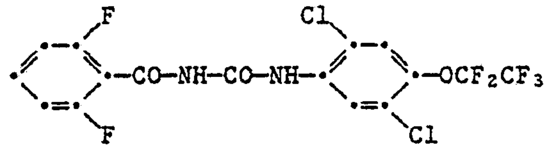

,

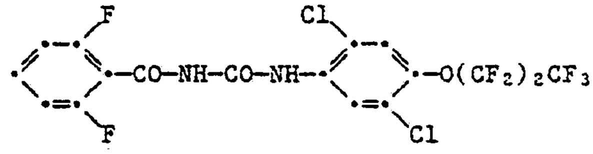

,

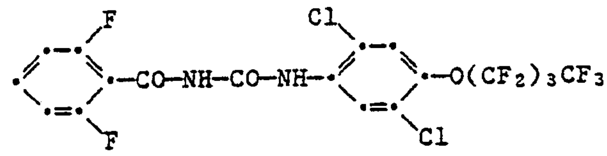

,

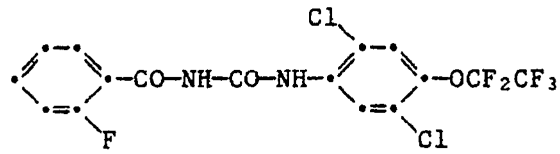

,

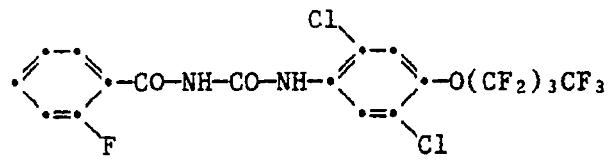

,

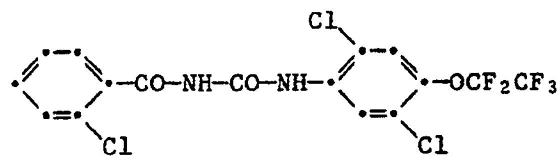

,

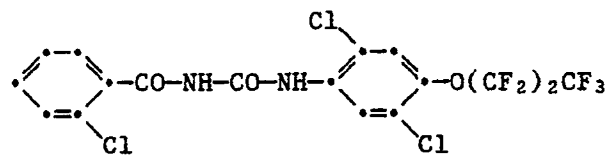

,

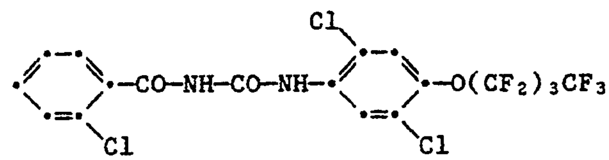

,

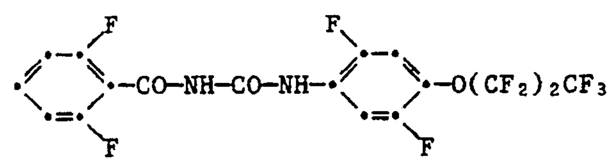

,

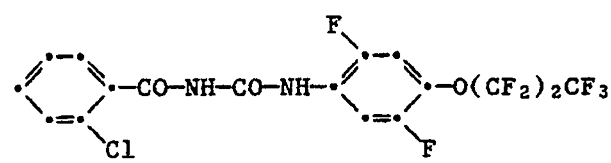

,

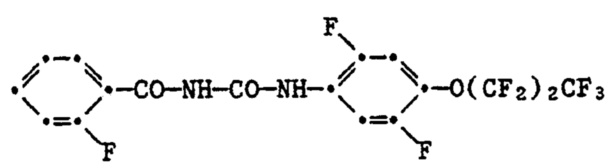

,

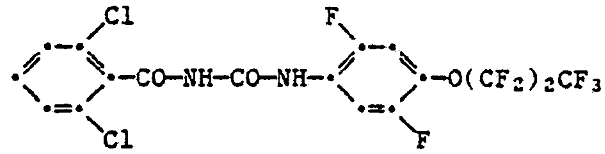 und

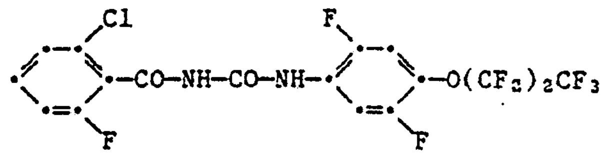 .

5. Verfahren zur Herstellung einer Verbindung der Formel I

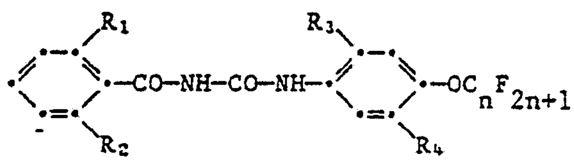 (I),

worin $R_1$ für Wasserstoff, Fluor, Chlor oder Brom ; $R_2$ für Fluor oder Chlor ; $R_3$ und $R_4$ je für Fluor, Chlor oder Brom ; und n für 2, 3, 4, 5, 6, 7 oder 8 stehen, mit der Massgabe, dass $R_4$ Fluor oder Chlor bedeutet, wenn gleichzeitig $R_3$ für Fluor und n für die Zahl 2 stehen,

a) ein Anilin der Formel II

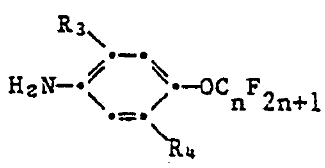 (II)

mit einem Benzoylisocyanat der Formel III

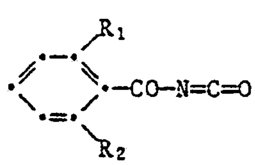 (III),

oder

b) ein Isocyanat der Formel IV

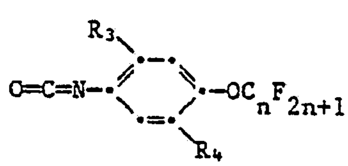 (IV)

mit einem Benzamid der Formel V

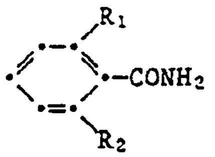

(V),

oder

c) ein Anilin der Formel II mit einem Urethan der Formel VI

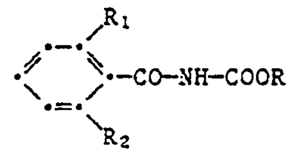

(VI)

umsetzt, wobei in den Formeln II bis VI die Symbole $R_1$, $R_2$, $R_3$, $R_4$ und n die angegebene Bedeutung haben und R für einen gegebenenfalls halogenierten $C_1$-$C_8$-Alkylrest steht.

6. Schädlingsbekämpfungsmittel, welches als aktive Komponente mindestens eine Verbindung der Formel I

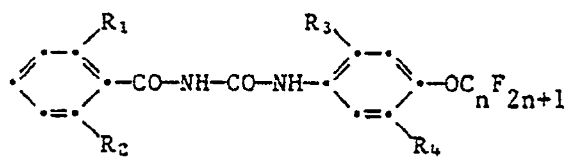

(I)

enthält, worin $R_1$ für Wasserstoff, Fluor, Chlor oder Brom ; $R_2$ für Fluor oder Chlor ; $R_3$ und $R_4$ je für Fluor, Chlor oder Brom ; und n für 2, 3, 4, 5, 6, 7 oder 8 stehen, mit der Massgabe, dass $R_4$ Fluor oder Chlor bedeutet, wenn gleichzeitig $R_3$ für Fluor und n für die Zahl 2 stehen, zusammen mit geeigneten Trägern und/oder Zuschlagstoffen.

7. Schlädlingsbekämpfungsmittel gemäss Anspruch 6, welches als aktive Komponente mindestens eine Verbindung der Formel I enthält, worin $R_1$ für Wasserstoff Fluor oder Chlor ; $R_2$ für Fluor oder Chlor ; $R_3$ für Fluor oder Chlor ; $R_4$ für Fluor, Chlor oder Brom ; und n für 2, 3, 4, 5, 6 oder 7 stehen.

8. Schädlingsbekämpfungsmittel gemäss Anspruch 7, welches als aktive Komponente mindestens eine Verbindung der Formel I enthält, worin $R_1$ für Wasserstoff oder Fluor ; $R_2$ für Fluor oder Chlor ; $R_3$ und $R_4$ je für Chlor ; und n für 2, 3 oder 4 stehen.

9. Verwendung einer Verbindung der Formel I

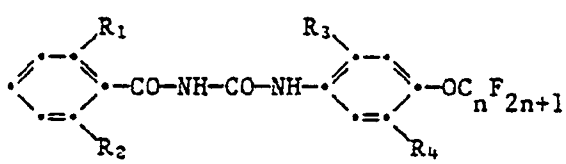

(I),

worin $R_1$ für Wasserstoff, Fluor, Chlor oder Brom ; $R_2$ für Fluor oder Chlor ; $R_3$ und $R_4$ je für Fluor, Chlor oder Brom ; und n für 2, 3, 4, 5, 6, 7 oder 8 stehen, mit der Massgabe, dass $R_4$ Fluor oder Chlor bedeutet, wenn gleichzeitig $R_3$ für Fluor und n für die Zahl 2 stehen, zur Bekämpfung von Schädlingen an Tieren und Pflanzen.

10. Verwendung gemäss Anspruch 9 einer Verbindung der Formel I zur Bekämpfung von Insekten und Arachniden.

11. Verwendung gemäss Anspruch 10 einer Verbindung der Formel I zur Bekämpfung larvaler Stadien von pflanzenschädigenden Insekten.

12. Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel I

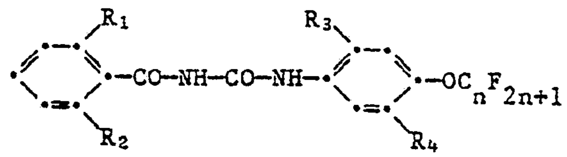 (I),

worin $R_1$ für Wasserstoff, Fluor, Chlor oder Brom ; $R_2$ für Fluor oder Chlor ; $R_3$ und $R_4$ je für Fluor, Chlor oder Brom ; und n für 2, 3, 4, 5, 6, 7 oder 8 stehen, mit der Massgabe, dass $R_4$ Fluor oder Chlor bedeutet, wenn gleichzeitig $R_3$ für Fluor und n für die Zahl 2 stehen in Kontakt bringt.

13. Verbindungen der Formel VII

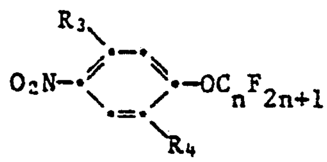 (VII),

worin $R_3$ und $R_4$ je Fluor, Chlor oder Brom bedeuten, und n für 2, 3, 4, 5, 6, 7 oder 8 steht, mit der Massgabe, dass $R_4$ Fluor oder Chlor bedeutet, wenn gleichzeitig $R_3$ für Fluor und n für die Zahl 2 stehen.

14. Verbindungen der Formel VII gemäss Anspruch 13, worin $R_3$ Fluor oder Chlor und $R_4$ Fluor, Chlor oder Brom bedeuten, und n für 2, 3, 4, 5, 6 oder 7 steht.

15. Verbindungen der Formel VII gemäss Anspruch 14, worin $R_3$ und $R_4$ je Chlor bedeuten, und n für 2, 3 oder 4 steht.

16. Die Verbindungen gemäss Anspruch 15 der Formeln

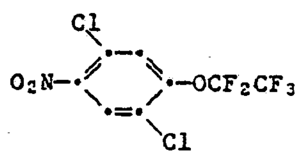 ,

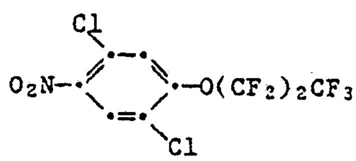 ,

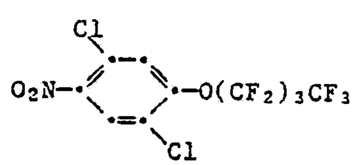 und

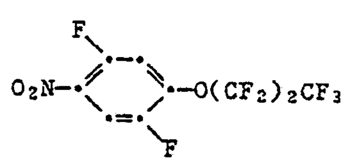 .

17. Verbindungen der Formel II

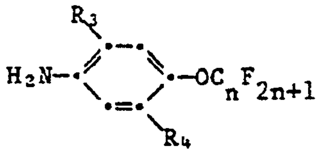

(II),

worin $R_3$ und $R_4$ je Fluor, Chlor oder Brom bedeuten, und n für 2, 3, 4, 5, 6, 7 oder 8 steht, mit der Massgabe, dass $R_4$ Fluor oder Chlor bedeutet, wenn gleichzeitig $R_3$ für Fluor und n für die Zahl 2 stehen.

18. Verbindungen der Formel II gemäss Anspruch 17, worin $R_3$ Fluor oder Chlor und $R_4$ Fluor, Chlor oder Brom bedeuten, und n für 2, 3, 4, 5, 6 oder 7 steht.

19. Verbindungen der Formel II gemäss Anspruch 18, worin $R_3$ und $R_4$ je Chlor bedeuten, und n für 2, 3 oder 4 steht.

20. Die Verbindungen gemäss Anspruch 19 der Formeln

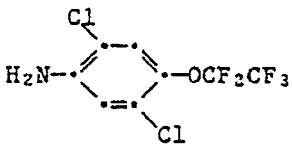

,

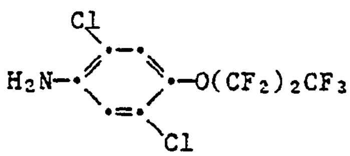

,

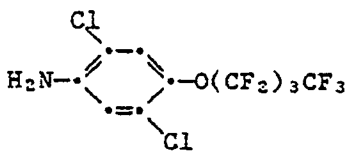

und

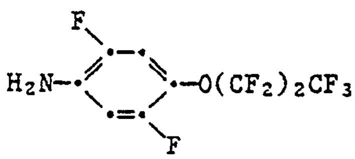

.

## Claims

1. Compounds of formula I

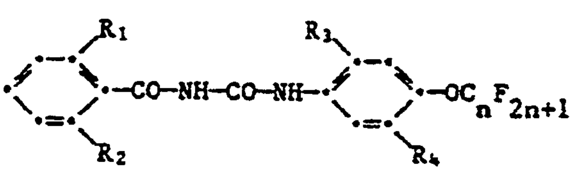

(I),

in which $R_1$ is hydrogen, fluorine, chlorine or bromine ; $R_2$ is fluorine or chlorine ; each of $R_3$ and $R_4$ is fluorine, chlorine or bromine ; and n is 2, 3, 4, 5, 6, 7 or 8, with the proviso that $R_4$ is fluorine or chlorine when $R_3$ is fluorine and, simultaneously, n is the number 2.

2. Compounds of formula I according to claim 1, in which $R_1$ is hydrogen, fluorine or chlorine ; $R_2$ is fluorine or chlorine ; $R_3$ is fluorine or chlorine ; $R_4$ is fluorine, chlorine or bromine ; and n is 2, 3, 4, 5, 6 or 7.

3. Compounds of formula I according to claim 2, in which $R_1$ is hydrogen or fluorine ; $R_2$ is fluorine or chlorine ; each of $R_3$ and $R_4$ is chlorine ; and n is 2, 3 or 4.

4. The compounds according to claim 3 of the formulae

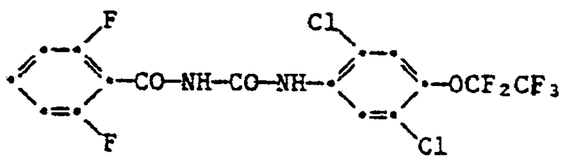 ,

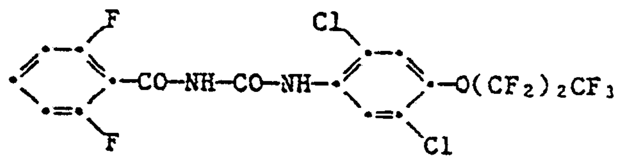 ,

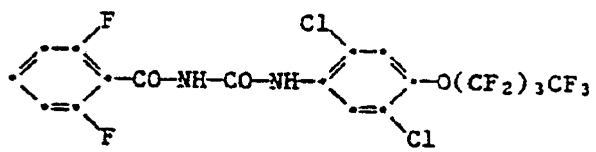 ,

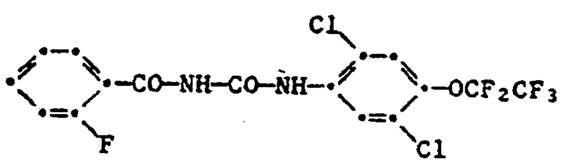 ,

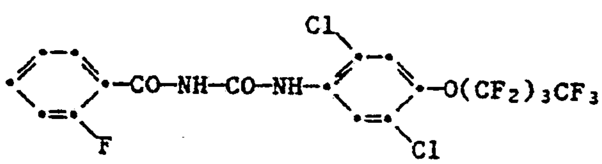 ,

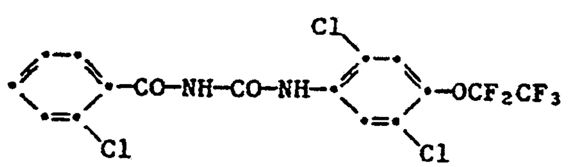 ,

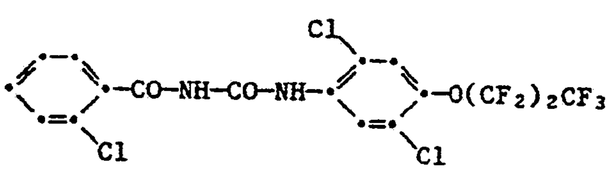 ,

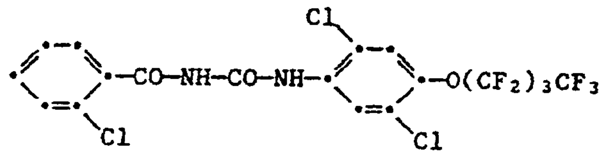 ,

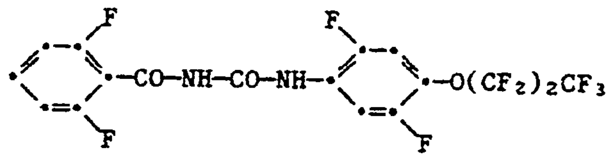 ,

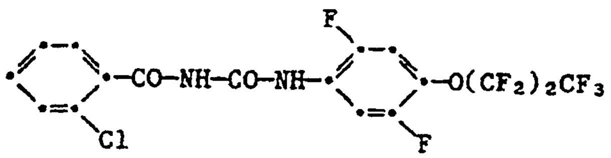 ,

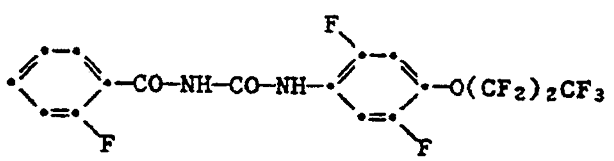 ,

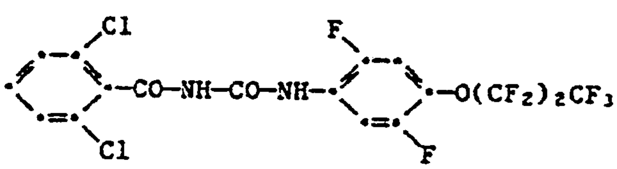 and

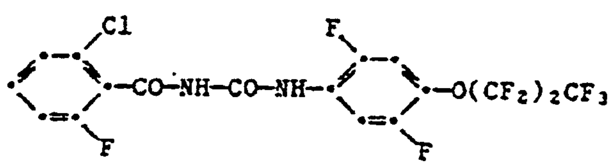 .

5. A process for the preparation of a compound of formula I

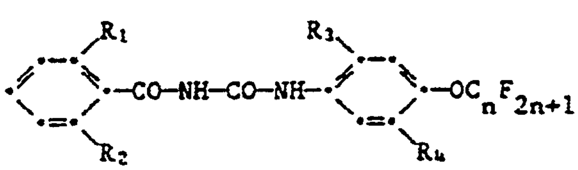 (I),

in which $R_1$ is hydrogen, fluorine, chlorine or bromine ; $R_2$ is fluorine or chlorine ; each of $R_3$ and $R_4$ is fluorine, chlorine or bromine ; and n is 2, 3, 4, 5, 6, 7 or 8, with the proviso that $R_4$ is fluorine or chlorine when $R_3$ is fluorine and, simultaneously, n is the number 2, which comprises

a) reacting an aniline of formula II

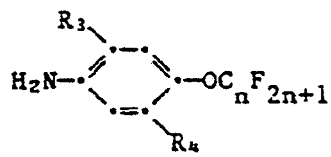

(II)

with a benzoyl isocyanate of formula III

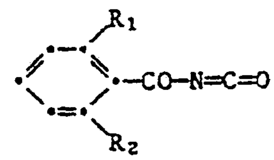

(III),

or

b) reacting an isocyanate of formula IV

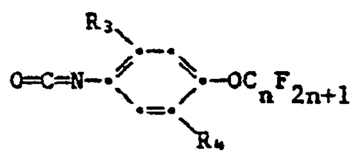

(IV)

with a benzamide of formula V

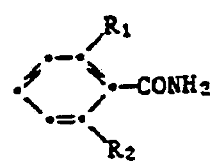

(V),

or

c) reacting an aniline of formula II with a urethane of formula VI

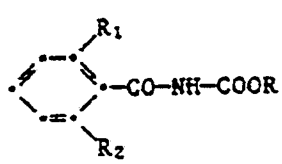

(VI)

the symbols $R_1$, $R_2$, $R_3$, $R_4$ and n in formulae II to VI having the meanings indicated and R being a $C_1$-$C_8$alkyl radical that is unsubstituted or is halogenated.

6. A pesticidal composition comprising as active component at least one compound of formula I

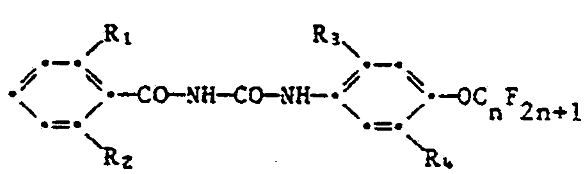

(I)

in which $R_1$ is hydrogen, fluorine, chlorine or bromine ; $R_2$ is fluorine or chlorine ; each of $R_3$ and $R_4$ is fluorine, chlorine or bromine ; and n is 2, 3, 4, 5, 6, 7 or 8, with the proviso that $R_4$ is fluorine or chlorine when $R_3$ is fluorine and, simultaneously, n is the number 2, together with suitable carriers and/or adjuvants.

7. A pesticidal composition according to claim 6, comprising as active component at least one compound

of formula I in which $R_1$ is hydrogen, fluorine or chlorine ; $R_2$ is fluorine or chlorine ; $R_3$ is fluorine or chlorine ; $R_4$ is fluorine, chlorine or bromine ; and n is 2, 3, 4, 5, 6 or 7.

8. A pesticidal composition according to claim 7, comprising as active component at least one compound of formula I in which $R_1$ is hydrogen or fluorine ; $R_2$ is fluorine or chlorine ; each of $R_3$ and $R_4$ is chlorine ; and n is 2, 3 or 4.

9. The use of a compound of formula I

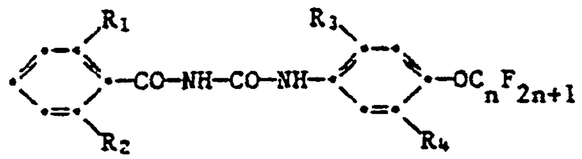

(I),

in which $R_1$ is hydrogen, fluorine, chlorine or bromine ; $R_2$ is fluorine or chlorine ; each of $R_3$ and $R_4$ is fluorine, chlorine or bromine ; and n is 2, 3, 4, 5, 6, 7 or 8, with the proviso that $R_4$ is fluorine or chlorine when $R_3$ is fluorine and, simultaneously, n is the number 2, for controlling pests in and on animals and plants.

10. The use according to claim 9 of a compound of formula I for controlling insects and arachnids.

11. The use according to claim 10 of a compound of formula I for controlling larval stages of plant-destructive insects.

12. A method of controlling pests in and on animals and plants, which comprises bringing the pests, in their various stages of development, into contact with a compound of formula I

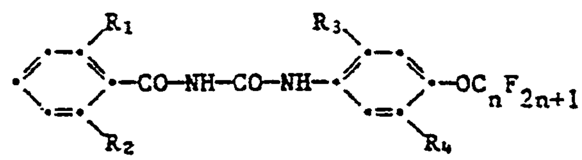

(I),

in which $R_1$ is hydrogen, fluorine, chlorine or bromine ; $R_2$ is fluorine or chlorine ; each of $R_3$ and $R_4$ is fluorine, chlorine or bromine ; and n is 2, 3, 4, 5, 6, 7 or 8, with the proviso that $R_4$ is fluorine or chlorine when $R_3$ is fluorine and, simultaneously, n is the number 2.

13. Compounds of formula VII

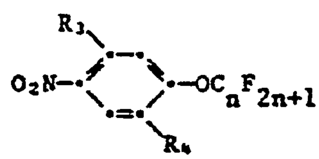

(VII),

in which each of $R_3$ and $R_4$ is fluorine, chlorine or bromine, and n is 2, 3, 4, 5, 6, 7 or 8, with the proviso that $R_4$ is fluorine or chlorine when $R_3$ is fluorine and, simultaneously, n is the number 2.

14. Compounds of formula VII according to claim 13, in which $R_3$ is fluorine or chlorine and $R_4$ is fluorine, chlorine or bromine, and n is 2, 3, 4, 5, 6 or 7.

15. Compounds of formula VII according to claim 14, in which each of $R_3$ and $R_4$ is chlorine and n is 2, 3 or 4.

16. The compounds according to claim 15 of formulae

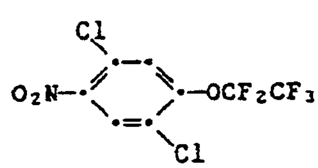

,

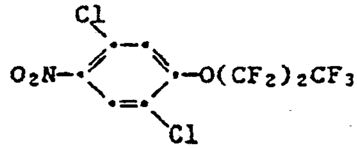

,

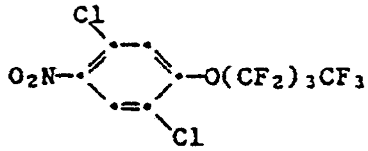

and

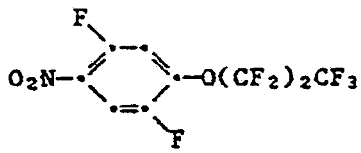

.

17. Compounds of formula II

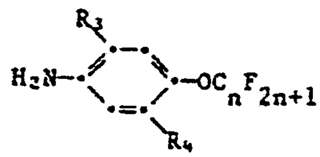

(II),

in which each of $R_3$ and $R_4$ is fluorine, chlorine or bromine, and n is 2, 3, 4, 5, 6, 7 or 8, with the proviso that $R_4$ is fluorine or chlorine when $R_3$ is fluorine and, simultaneously, n is the number 2.

18. Compounds of formula II according to claim 17, in which $R_3$ is fluorine or chlorine and $R_4$ is fluorine, chlorine or bromine, and n is 2, 3, 4, 5, 6 or 7.

19. Compounds of formula II according to claim 18, in which each of $R_3$ and $R_4$ is chlorine, and n is 2, 3 or 4.

20. The compounds according to claim 19 of formulae

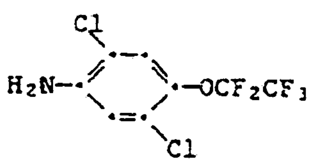

,

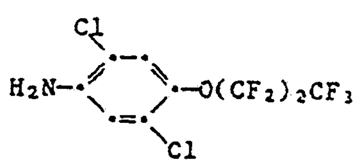

,

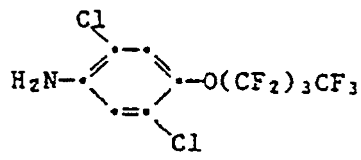

and

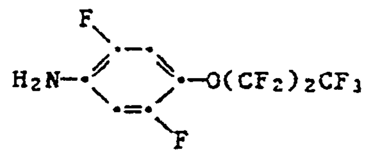

.

## Revendications

1. Composés de formule I

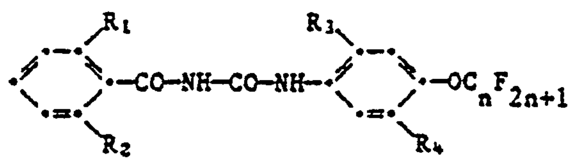

(I),

dans laquelle $R_1$ est l'hydrogène, le fluor, le chlore ou le brome ; $R_2$ est le fluor ou le chlore ; $R_3$ et $R_4$ sont le fluor, le chlore ou le brome ; et n représente 2, 3, 4, 5, 6, 7 ou 8, avec la condition que $R_4$ représente le fluor ou le chlore quand simultanément $R_3$ est le fluor et n est le nombre 2.

2. Composés de formule I selon la revendication 1, dans lesquels $R_1$ est l'hydrogène, le fluor ou le chlore; $R_2$ le fluor ou le chlore ; $R_3$ le fluor ou le chlore ; $R_4$ le fluor, le chlore ou le brome, et n est 2, 3, 4, 5, 6 ou 7.

3. Composés de formule I selon la revendication 2, dans laquelle $R_1$ est l'hydrogène ou le fluor ; $R_2$ est le fluor ou le chlore ; $R_3$ et $R_4$ représentent chacun le chlore ; et n représente 2, 3 ou 4.

4. Composés selon la revendication 3, de formules

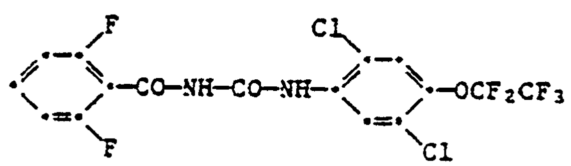

,

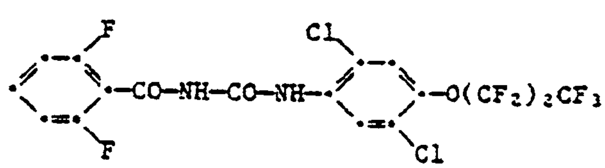

,

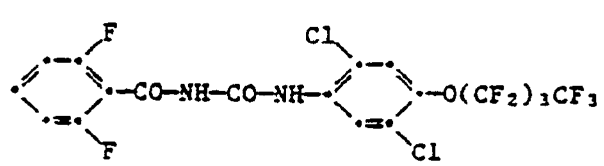

,

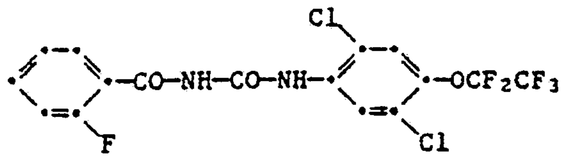 ,

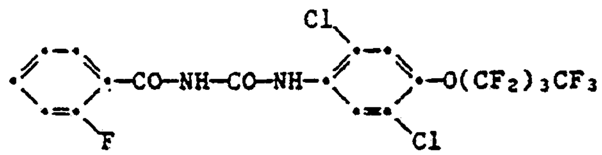 ,

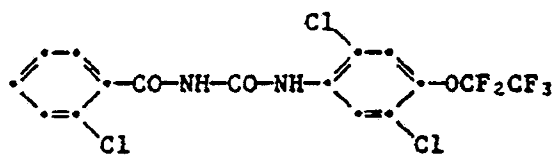 ,

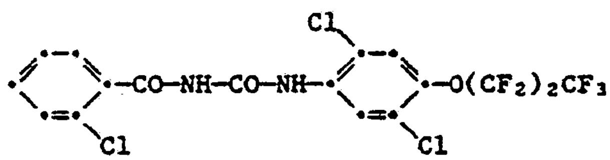 ,

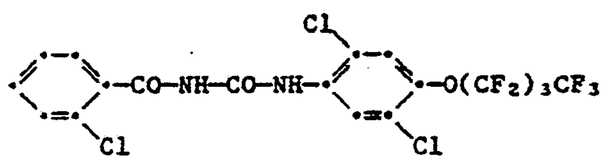 ,

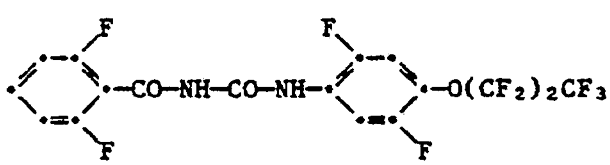 ,

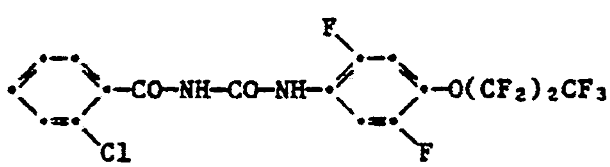 ,

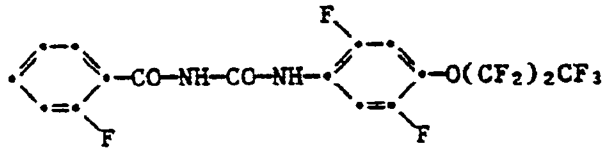 ,

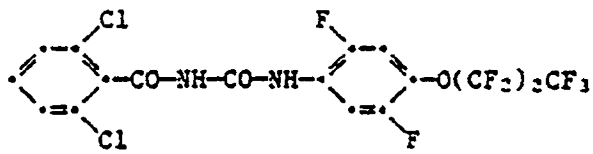 et

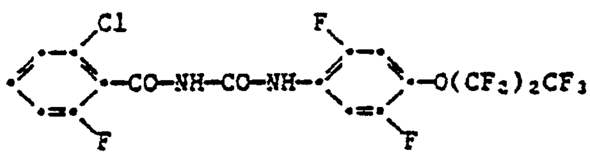 .

5. Procédé de préparation d'un composé de formule

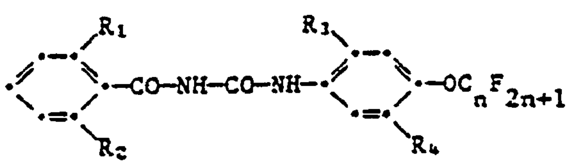 (I),

dans laquelle $R_1$ est l'hydrogène, le fluor, le chlore ou le brome ; $R_2$ est le fluor ou le chlore ; $R_3$ et $R_4$ sont le fluor, le chlore ou le brome ; et n représente 2, 3, 4, 5, 6, 7 ou 8, avec la condition que $R_4$ représente le fluor ou le chlore quand simultanément $R_3$ est le fluor et n est le nombre 2, caractérisé en ce qu' on fait réagir

a) une aniline de formule II

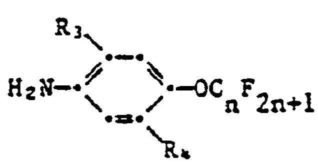 (II)

avec un benzoylisocyanate de formule III

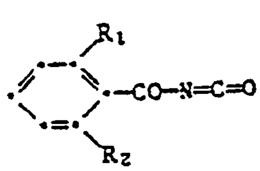 (III),

ou

b) un isocyanate de formule IV

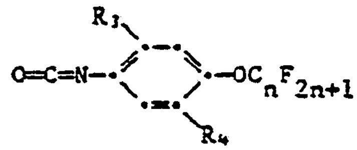

(IV)

avec un benzamide de formule V

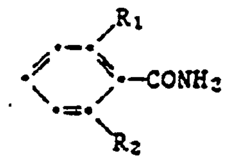

(V),

ou

c) une aniline de formule II avec un uréthane de formule VI

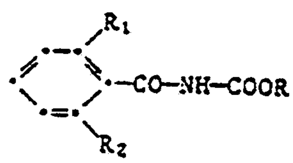

(VI)

dans les formules II à VI les symboles $R_1$, $R_2$, $R_3$, $R_4$ et n ont la signification indiquée, et R est un reste alkyle $C_1$-$C_8$ halogéné le cas échéant.

6. Moyen de lutte antiparasitaire qui contient comme composant actif au moins un composé de formule I

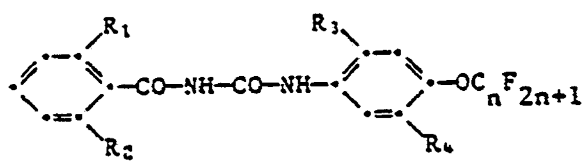

(I)

dans laquelle $R_1$ est l'hydrogène, le fluor, le chlore ou le brome ; $R_2$ est le fluor ou le chlore ; $R_3$ et $R_4$ sont le fluor, le chlore ou le brome ; et n représente 2, 3, 4, 5, 6, 7 ou 8, avec la condition que $R_4$ représente le fluor ou le chlore quand simultanément $R_3$ est le fluor et n est le nombre 2, avec des véhicules appropriés et/ ou des additifs.

7. Moyen de lutte antiparasitaire selon la revendication 6 qui contient comme composant actif au moins un composé de formule I dans laquelle $R_1$ est l'hydrogène, le fluor ou le chlore ; $R_2$ est le fluor ou le chlore ; $R_3$ est le fluor ou le chlore ; $R_4$ est le fluor, le chlore ou le brome ; et n représente 2, 3, 4, 5, 6 ou 7.

8. Moyen de lutte antiparasitaire selon la revendication 7, qui contient comme composant actif au moins un composé de formule I dans laquelle $R_1$ est l'hydrogène ou le fluor ; $R_2$ est le fluor ou le chlore ; $R_3$ et $R_4$ représentent chacun le chlore ; et n représente 2, 3 ou 4.

9. Utilisation d'un composé de formule I

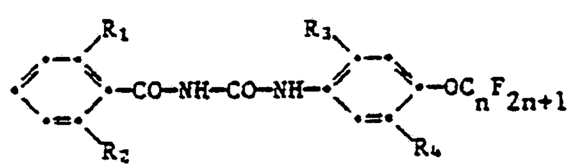

(I),

dans laquelle $R_1$ est l'hydrogène, le fluor, le chlore ou le brome ; $R_2$ est le fluor ou le chlore ; $R_3$ et $R_4$ sont le fluor, le chlore ou le brome ; et n représente 2, 3, 4, 5, 6, 7 ou 8, avec la condition que $R_4$ représente le fluor

ou le chlore quand simultanément $R_3$ est le fluor et n est le nombre 2, pour combattre les parasites des animaux et des plantes.

10. Utilisation selon la revendication 9 d'un composé de formule I pour combattre les insectes et les arachnides.

11. Utilisation selon la revendication 10 d'un composé de formule I pour combattre les stades larvaires des insectes nuisibles des plantes.

12. Procédé de lutte contre les parasites des animaux et des plantes caractérisé en ce qu'on met en contact les parasites à leurs différents stades de développement avec un composé de formule I

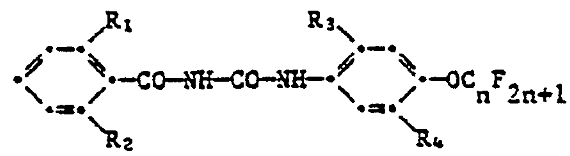 (I),

dans laquelle $R_1$ est l'hydrogène, le fluor, le chlore ou le brome ; $R_2$ est le fluor ou le chlore ; $R_3$ et $R_4$ sont le fluor, le chlore ou le brome ; et n représente 2, 3, 4, 5, 6, 7 ou 8, avec la condition que $R_4$ représente le fluor ou le chlore quand simultanément $R_3$ est le fluor et n est le nombre 2.

13. Composés de formule VII

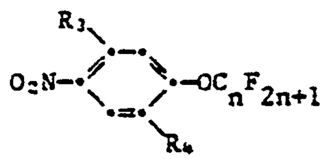 (VII),

dans laquelle $R_3$ et $R_4$ sont le fluor, le chlore ou le brome ; et n représente 2, 3, 4, 5, 6, 7 ou 8, avec la condition que $R_4$ représente le fluor ou le chlore quand simultanément $R_3$ est le fluor et n est le nombre 2.

14. Composés de formule VII selon la revendication 13, dans laquelle $R_3$ représente le fluor ou le chlore et $R_4$ le fluor, le chlore ou le brome, et n représente 2, 3, 4, 5, 6 ou 7.

15. Composés de formule VII selon la revendication 14, dans laquelle $R_3$ et $R_4$ sont tous deux le chlore et n représente 2, 3 ou 4.

16. Composés selon la revendication 15 de formules

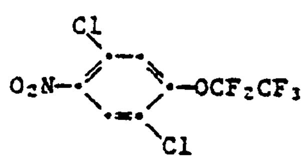 ,

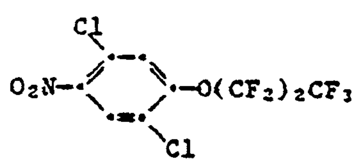 ,

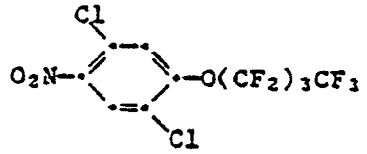 et

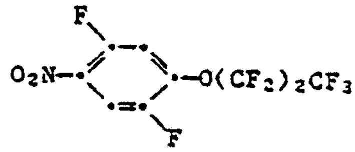

.

17. Composés de formule II

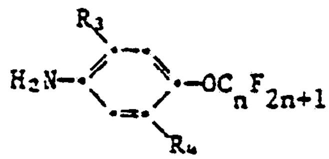

(II),

dans laquelle $R_3$ et $R_4$ sont le fluor, le chlore ou le brome ; et n représente 2, 3, 4, 5, 6, 7 ou 8, avec la condition que $R_4$ représente le fluor ou le chlore quand simultanément $R_3$ est le fluor et n est le nombre 2.

18. Composés de formule II selon la revendication 17, dans laquelle $R_3$ représente le fluor ou le chlore et $R_4$ représente le fluor, le chlore ou le brome, et n représente 2, 3, 4, 5, 6 ou 7.

19. Composés de formule II selon la revendication 18, dans laquelle $R_3$ et $R_4$ représentent chacun le chlore, et n représente 2, 3 ou 4.

20. Composés selon la revendication 19 de formules

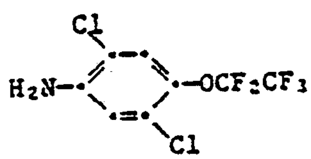

,

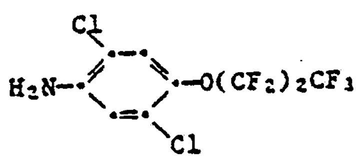

,

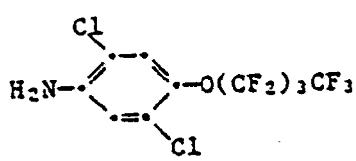

et

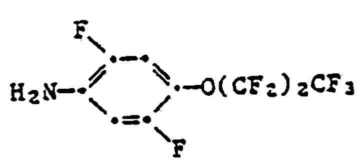

.